# EUROPEAN PATENT APPLICATION

(11) **EP 3 822 282 A1**
(43) Date of publication of application: **19.05.2021**
(21) Application number: 19209714.5
(22) Date of filing: 18.11.2019
(51) Int. Cl.: C07H 1/06, C07H 1/08, C07H 3/06

(54) **PROCESS FOR REMOVING AN ANTIFOAM AGENT FROM A SOLUTION COMPRISING A HUMAN MILK OLIGOSACCHARIDE AND RELATED COMPOSITIONS**

(71) Applicant: DuPont Nutrition Biosciences ApS, 1411 Copenhagen K (DK)
(72) Inventor: JÄRVINEN, Juho, 02460 Kantvik (FI); KOIVIKKO, Hannu, 02460 Kantvik (FI); KOPONEN, Antti, 02460 Kantvik (FI); KUUSISTO, Jyrki, 02460 Kantvik (FI); LEWANDOWSKI, Jari, 02460 Kantvik (FI); MENTUNEN, Tero, 02460 Kantvik (FI)
(74) Representative: DuPont EMEA

(57) **Abstract**

This specification relates to a process for preparing a purified human milk oligosaccharide ("HMO") from an HMO-containing solution (e.g., a fermentation broth) by a process comprising removal of an antifoam agent, and a product of such a process.

## Description

### FIELD

This specification relates to preparing a purified human milk oligosaccharide ("HMO") from an HMO-containing solution (*e.g.,* a fermentation broth) by a process comprising removal of an antifoam agent, and a product of such a process.

### BACKGROUND

Human milk oligosaccharides are important for nutrition and therapeutics. HMOs include, for example, 2'-fucosyllactose ("2'-FL"), 3-fucosyllactose ("3-FL"), lacto-N-tetraose ("LNT"), 6'-sialyllactose ("6'-SL"), 3'-sialyllactose ("3'-SL"), difucosyllactose ("DiFL" or "LDFT"), lacto-N-neotetraose ("LNnT"), lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, lacto-N-triose, lacto-N-neofucopentaose, lacto-N-neofucopentaose, lacto-N-difucohexaose, 6'-galactosyllactose, 3'-galactosyllactose, lacto-N-hexaose and lacto-N-neohexaose. Many HMOs in human breast milk are fucosylated, unlike oligosaccharides produced by, for example, dairy animals. The most abundant HMO in human breast milk is 2'-FL.

Many recent approaches for synthesizing HMOs involve microbial fermentation processes, which produce HMOs (such as 2'-FL, 3-FL, LNT, 3'-SL and 6'-SL) from lactose. In such a process, a given HMO is synthesized by cultured microorganisms, such as recombinant *E. coli.* The HMO is then isolated from the broth of biomolecules produced by the culture through a series of purification processes. While there has been success with this approach, the fermentation processes generally produce a complex product mixture which includes, besides the desired HMO(s), other ingredients, such as monovalent and divalent salts, lactose, oligosaccharides, monosaccharides, amino acids, polypeptides, proteins, organic acids, nucleic acids, processing aids, etc. Consequently, there continues to be a need for effective, reliable and economically feasible downstream purification processes that provide a useable purified HMO product.

Aerobic submerged fermentation generally relies on aeration to supply oxygen required by the microorganisms to grow and produce the product of interest. The introduction of air into a fermentation broth to provide oxygen for the microorganisms typically generates foam. Mixing of the fermentation broth also generates foam. The presence of such foam can negatively impact performance, including reduction of fermenter working volume or productivity, and a risk of contamination associated with a "foam out" (*i.e.,* the production of a foam column or foam head above a liquid fermentation broth that has sufficient height to exit the fermentation vessel through venting, pipes, etc.).

Additives, such as antifoam or defoamers, are commonly used to mitigate foam formation during fermentation. But these additives, in turn, can negatively impact subsequent processes to recover the desired HMO product. For example, membrane-based separation processes and ion exchange operations can be negatively affected by such additives due to fouling and the consequent slowing of the process. Thus, depending on the end-use application of the recovered HMO product, the additives (*e.g.,* antifoam agents) employed during the production process may need to be removed.

HMOs may be incorporated into a food (*e.g.,* human or pet food), dietary supplement or medicine. HMOs are particularly useful in, for example, infant formula. Thus, there is need for HMOs that are substantially pure.

In current processes for manufacturing HMOs, removal of antifoam agent requires heating and cooling steps that consume resources like time, energy and water. While the concentration of precipitated antifoam agent can be reduced, solubilized antifoam agent can remain and ultimately end up the final product. Accordingly, a need continues to exist for reliable and economically and environmentally feasible processes to remove antifoam agents to provide an HMO product of high quality and purity.

### SUMMARY

Briefly, this specification generally provides, in part, a process for making a purified human milk oligosaccharide ("HMO") derived from a fermentation process. The process comprises precipitating an antifoam agent in a solution comprising the HMO and the antifoam agent by heating the solution to a precipitation temperature of from 40 to 65°C, and filtering precipitated antifoam agent from the HMO solution at the precipitation temperature. Here, the precipitation temperature is within 5°C of a temperature at which the HMO solution exhibits maximum turbidity.

This specification also provides, in part, a purified HMO (or mixture of HMOs) obtained by an above-referenced process.

This specification also provides, in part, a process for making a food, dietary supplement or medicine. The process comprises preparing a purified HMO (or mixture of HMOs) according to an above-described process, and mixing the purified HMO (or HMO mixture) with an ingredient suitable for the food, dietary supplement or medicine. This specification also provides, in part, a food, dietary supplement or medicine prepared by such a process.

This specification also provides, in part, a process for making an infant formula. The process comprises preparing a purified HMO (or mixure of HMOs) according to an above-described process, and mixing the purified HMO (or HMO mixture) with an infant formula ingredient. This specification also provides, in part, an infant formula prepared by such a process.

Further benefits of the teachings of this specification will be apparent to one skilled in the art from reading this specification.

### BRIEF DESCRIPTION OF THE FIGURE

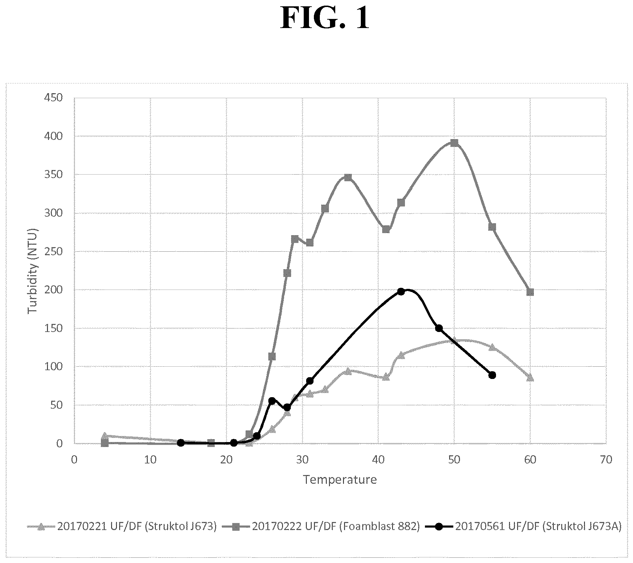

**Figure 1** is a graphical presentation of the turbidity curves of the various antifoam agents according to **Example 8.**

### DETAILED DESCRIPTION

This detailed description is intended to acquaint others skilled in the art with Applicant's invention, its principles, and its practical application so that others skilled in the art may adapt and apply Applicant's invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.

### Definitions

The term "Area%" refers to normalized peak area purity or concentration obtained using HPLC. This is a percentage of peak area relative to the total area of peaks.

The term "cloud point temperature" or "cloud point" refers to the cloud point temperature of an antifoam agent.

The terms "DiFL" or "LDFT" refer to difucosyllactose (also referred to as "lactodifucotetraose").

The term "DS" refers to a dry substance content expressed as percent by weight.

The term "2'-FL" refers to 2'-fucosyllactose (also referred to as "2'-O-fucosyllactose").

The term "3-FL" refers to 3-fucosyllactose (also referred to as "3-O-fucosyllactose").

The term "HMO" refers to human milk oligosaccharide.

The term "HPLC" refers to high performance liquid chromatography.

The term "ICUMSA" refers to "International Comission for Uniform Process of Sugar Analysis" sugar color grading system.

The term "LNnT" refers to lacto-N-neotetraose.

The term "LNT" refers to lacto-N-tetraose.

The term "NTU" refers to Nephelometric Turbidity Unit. This is a unit of turbidity measuring scattered light at 90 degrees from the incident light beam using white light.

The term "3 '-SL" refers to 3 '-sialyllactose (also referred to as "N-acetylneuraminyl-2-3-galactopyranosyl-1-4-glucopyranose").

The term "6'-SL" refers to 6'-sialyllactose.

### Human milk oligosaccharide

There are over 150 known human milk oligosaccharides generally present in human breast milk. A process described in this specification may be used to prepare a single purified HMO or a purified mixture of two or more HMOs.

In some embodiments, a process of this specification comprises preparing a purified HMO selected from fucosyllactoses (such as 2'-fucosyllactose ("2'-FL"), 3-fucosyllactose ("3-FL") or difucosyllactose ("DiFL" or "LDFT")), sialyllactoses (such as 3'-siayllactose ("3'-SL") or 6'-sialyllactose ("6'-SL")), lacto-N-tetraose ("LNT"), lacto-N-neotetraose ("LNnT"), lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, lacto-N -triose, lacto-N-neo fucopentaose, lacto-N-neofucopentaose, lacto-N- difucohexaose, 6'-galactosyllactose, 3'-galactosyllactose, lacto-N-hexaose or lacto-N-neohexaose. In some embodiments, a process of this specification comprises preparing a purified HMO mixture comprising one or more of the above-listed HMOs. In some embodiments, a process of this specification comprises preparing a purified HMO mixture comprising at least two of the above-listed HMOs.

In some embodiments, a process of this specification is used to prepare a purified HMO selected from a fucosyllactose (*e.g.,* 2'-FL, 3-FL or DiFL), a sialyllactose (*e.g.,* 3'-SL or 6'-SL), LNT or LNnT.

In some embodiments, a process of this specification is used to prepare a purified fucosyllactose (also referred to as "FL"). At room temperature and pressure, a fucosyllactose is typically a white to ivory coloured solid and soluble in water. In some embodiments, the purified fucosyllactose is 2'-FL. In some embodiments, the purified fucosyllactose is 3-FL. In some embodiments, a process of this specification is used to prepare a purified HMO mixture comprising a fucosyllactose. In some embodiments, a process of this specification is used to prepare a purified HMO mixture comprising 2'-FL, 3-FL or DiFL. In some embodiments, a process of this specification is used to prepare a purified HMO mixture comprising at least two fucosyllactoses. In some embodiments, a process of this specification is used to prepare a purified HMO mixture comprising 2'-FL and DiFL.

In some embodiments, a process of this specification comprises preparing a purified sialyllactose (also referred to as "SL"). In some embodiments, the purified sialyllactose is 3'-SL. In some embodiments, the purified sialyllactose is 6'-SL". In some embodiments, a process of this specification is used to make a purified HMO mixture comprising a sialyllactose. In some embodiments, a process of this specification is used to prepare a purified HMO mixture comprising 3'-SL or 6'-SL. In some embodiments, a process of this specification is used to prepare a purified HMO mixture comprising at least two sialyllactoses. In some embodiments, a process of this specification is used to prepare a purified HMO mixture comprising 3'-SL and 6'-SL.

In some embodiments, a process of this specification comprises preparing purified lacto-N-tetraose ("LNT"). In some embodiments, the process of this specification is used to make a purified HMO mixture comprising LNT.

In some embodiments, a process of this specification comprises preparing purified lacto-N-neotetraose ("LNnT"). In some embodiments, the process of this specification is used to make a purified HMO mixture comprising LNnT.

### HMO Solution

An "HMO solution" from which an HMO is purified in accordance with this specification generally comprises an aqueous medium. The aqueous medium comprises both the HMO and other ingredients, for example, monovalent and divalent salts, lactose, oligosaccharides, monosaccharides, amino acids, polypeptides, proteins, organic acids, nucleic acids, and the antifoam agent.

In some embodiments, the aqueous medium is water.

In some embodiments, the HMO is selected from 2'-FL, 3-FL, LNT, 6'-SL, 3'-SL, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, lacto-N-triose, lacto-N-neo fucopentaose, lacto-N-neofucopentaose, lacto-N-difucohexaose, 6'-galactosyllactose, 3'-galactosyllactose, lacto-N-hexaose, and lacto-N-neohexaose.

In some embodiments, the HMO is a fucosyllactose.

In some embodiments, the HMO is 2'-FL.

In some embodiments, the HMO is 3-FL.

In some embodiments, the HMO is DiFL.

In some embodiments, the HMO is a sialyllactose.

In some embodiments, the HMO is 3'-SL.

In some embodiments, the HMO is 6'-SL.

In some embodiments, the HMO is LNnT.

In some embodiments, the HMO is LNT.

In some embodiments, the HMO solution comprises at least two HMOs. In some embodiments, the HMO solution comprises at least three HMOs. In some embodiments, the HMO solution comprises at least four HMOs. In some embodiments, the HMO solution comprises at least five HMOs.

In some embodiments, the HMO solution comprises two or more HMOs selected from fucosyllactoses, sialyllactoses, LNnT and LNT. In some such embodiments, the fucosyllactoses are selected from 2'-FL, DiFL and 3-FL; and the sialyllactoses are selected from 3'-SL and 6'-SL.

In some embodiments, the HMO solution comprises 2'-FL and 3-FL.

In some embodiments, the HMO solution comprises 2'-FL and DiFL.

In some embodiments, the HMO solution comprises 3'-SL and 6'-SL.

Typically, the HMO solution further comprises one or more ingredients in addition to the HMO(s) to be purified. Such other ingredients may include, for example, monovalent and divalent salts, lactose, oligosaccharides, monosaccharides, amino acids, polypeptides, proteins, organic acids, nucleic acids, etc.

In some embodiments, the HMO solution comprises (in addition to the HMO(s) to be purified) one or more additional HMOs and/or one or more other types of carbohydrates.

In some embodiments, the HMO solution comprises (in addition to the HMO(s) to be purified) one or more oligosaccharides.

In some embodiments, the HMO solution comprises (in addition to the HMO(s) to be purified) one or more additional HMOs.

In some embodiments, the HMO solution comprises (in addition to the HMO(s) to be purified) one or more additional HMOs selected from 2'-FL, 3-FL, LNT, 6'-SL, 3'-SL, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, lacto-N -triose, lacto-N-neo fucopentaose, lacto-N - neofucopentaose, lacto-N- difucohexaose, 6'-galactosyllactose, 3'-galactosyllactose, lacto-N - hexaose, and lacto-N -neohexaose.

In some embodiments, the HMO solution comprises (in addition to the HMO(s) to be purified) 2'-O-fucosyl lactulose.

In some embodiments, the HMO solution comprises (in addition to the HMO(s) to be purified) DiFL.

In some embodiments, the HMO solution comprises (in addition to the HMO(s) to be purified) lactose.

In some embodiments, the HMO solution comprises (in addition to the HMO(s) to be purified) lactulose.

In some embodiments, the HMO solution comprises (in addition to the HMO(s) to be purified) one or more monosaccharides.

In some embodiments, the HMO solution comprises (in addition to the HMO(s) to be purified) fucose.

In some embodiments, the HMO solution comprises (in addition to the HMO(s) to be purified and the second carbohydrate) glucose.

In some embodiments, the HMO solution comprises (in addition to the HMO(s) to be purified) galactose.

In some embodiments, the HMO solution comprises (in addition to the HMO(s) to be purified) one or more monovalent salts.

In some embodiments, the HMO solution comprises (in addition to the HMO(s) to be purified) one or more divalent salts.

In some embodiments, the HMO solution comprises (in addition to the HMO(s) to be purified) one or more amino acids.

In some embodiments, the HMO solution comprises (in addition to the HMO(s) to be purified) one or more proteins.

In some embodiments, the HMO solution comprises (in addition to the HMO(s) to be purified) one or more organic acids.

In some embodiments, the HMO solution comprises (in addition to the HMO(s) to be purified) one or more nucleic acids.

In some embodiments, the HMO solution comprises (or is derived in whole or in part from) a product of a fermentation. In some such embodiments, the HMO solution is (or derived in whole or in part from) the product of a fermentation used to make the HMO(s) to be purified. In some such embodiments, the other carbohydrate(s) in the solution is/are from the culture medium used in the fermentation and/or formed during and/or after the fermentation. In some embodiments, the fermentation comprises culturing, in an aqueous culture medium comprising a carbohydrate (such as lactose and/or fucose), a recombinant microorganism comprising at least one recombinant polynucleotide sequence encoding an enzyme capable of producing an HMO. The product of the fermentation process maybe referred to as a fermentation "product" or "broth." The product typically comprises many ingredients in addition to the HMO(s) to be purified, including, for example, monovalent and divalent salts, lactose, oligosaccharides, monosaccharides, amino acids, polypeptides, proteins, organic acids, nucleic acids, etc.

In some embodiments, the HMO(s) to be purified is a fucosyllactose, and the HMO solution comprises (or is derived in whole or in part from) a product of a fermentation process wherein the fermentation process comprises culturing, in an aqueous culture medium comprising a carbohydrate (such as lactose and/or fucose), a recombinant microorganism comprising a recombinant polynucleotide sequence encoding an α-1,2-fucosyl transferase (EC 2.4.1.69) or α-1,3-fucosyl transferase (EC 2.4.1.214).

In general, when the HMO solution comprises (or is derived in whole or in part from) a product of a fermentation process, the process of this specification comprises one or more process steps wherein the cell biomass of the microorganisms used in the fermentation is separated from the fermentation product.

Cell biomass may be separated from a fermentation product using, for example, filtration, centrifugation, sedimentation and/or other process suitable for removing cell biomass.

In some embodiments, separation of microorganisms from a fermentation product comprises ultrafiltration (also referred to as "UF"). Ultrafiltration can also be particularly beneficial to, for example, remove large biomolecules, such as endotoxins, proteins, nucleic acids and lipopolysaccharides.

In some embodiments, the ultrafiltration is carried out using a cross-flow filtration. The polymeric membrane configuration used can be, for example, a spiral wound, hollow fiber or plate and frame unit. The ultrafiltration can also be carried out with tubular or ceramic disc membranes. Typically, the ultrafiltration membrane pore size can be chosen from about 0.1 to about 0.001 µm, or from about 200 kD to about 1 kD.

In some embodiments, separation of microorganisms from a fermentation product comprises cross-flow microfiltration (also referred to as "MF"). Typically, the MF membrane pore size is from about 0.1 µm to about 3 µm. The polymeric membrane configuration used can be, for example, a spiral wound, hollow fiber or plate and frame unit. The cross-flow microfiltration can also be carried out with ceramic tubular or ceramic disc membranes. Furthermore, MF membranes made of steel can be used.

In some embodiments, separation of microorganisms from a fermentation product comprises centrifugation. Typically, such a centrifugation is carried out using disc stack separator reaching from about 3000 to about 20000 G-force. The clarified solution can be further purified with, for example, filtration technologies to obtain liquid essentially free of microbes.

In some embodiments, the cell biomass removal is carried out at a temperature of no greater than about 18°C. In some embodiments, the cell biomass removal is carried out at a temperature of no greater than 16°C. In some embodiments, the cell biomass removal is carried out at a temperature of less than 16°C. In some embodiments, the cell biomass removal is carried out at a temperature of no greater than 15°C. In some embodiments, the cell biomass removal is carried out at a temperature of less than 15 °C. In some embodiments, the cell biomass removal is carried out at a temperature of no greater than 10°C. In some embodiments, the cell biomass removal is carried out at a temperature of less than 10°C. In some embodiments, the cell biomass removal is carried out at a temperature of no greater than 9°C. In some embodiments, the cell biomass removal is carried out at a temperature of less than 9°C. In some embodiments, the cell biomass removal is carried out at a temperature of no greater than 8°C. In some embodiments, the cell biomass removal is carried out at a temperature of less than 8°C. In some embodiments, the cell biomass removal is carried out at a temperature of no greater than 7°C. In some embodiments, the cell biomass removal is carried out at a temperature of less than 7°C. In some embodiments, the cell biomass removal is carried out at a temperature of no greater than 6°C. In some embodiments, the cell biomass removal is carried out at a temperature of less than 6°C. In some embodiments, the cell biomass removal is carried out at a temperature of no greater than 5°C. In some embodiments, the cell biomass removal is carried out at a temperature of less than 5°C.

### Antifoam Agent

Antifoam agents are chemical additives that reduce or hinder foam formation or break down a foam that has already formed. In general, antifoam agents are surfactants. Antifoam agents are widely used in submerged fermentation processes to control the foam build up in fermenters. Antifoam agents may be either natural or synthetic. Antifoam agents may be, for example, silicone-based, polyethers, natural oils or polyalcohols. In some embodiments, the antifoam agent displays a cloud point. Such antifoam agents include, for example, poly(alkylene glycol) ("PAG") based antifoam agents, such as ethylene oxide/propylene oxide ("EO/PO") block co-polymers; polyalcohols based on EO/PO block copolymers, and polyethers of ethylene and propylene oxides; and fatty acid ester ("FE") based antifoam agents, such as alkoxylated fatty acid esters.

In some embodiments, antifoam agent is based on fatty acid esters. In some embodiments, the antifoam agent comprises one or more alkoxylated fatty acid esters on a vegetable base such as, for example, STRUKTOL J673 or STRUKTOL J673A (Scill + Seilacher "Struktol" GmbH, Hamburg, Germany) or non-mineral oils such as, for example, FOAM BLAST 882 (DyStar Singapore Pte Ltd, Singapore). Antifoam agents may be used alone or as a mixture with other antifoam agents.

In some embodiments, the antifoam agent is non-ionic.

Antifoam agents may be added to the fermentation vessel before or at the start of the fermentation to prevent foam forming. Alternatively or additionally, the antifoam agent is added during the fermentation to prevent foam from forming and/or destroy foam that has already formed. In some instances, an antifoam agent added to the fermentation vessel during the fermentation is referred to as a defoamer. In some instances, an antifoam agent may be characterized as a "foam inhibitor" and/or a "foam breaker." In practice, most foam-dispersing chemicals can serve either role.

The antifoam agent generally must be safe to the microorganisms used in the fermentation. The antifoam agent also generally must not react with the desired HMO product or impact product colour or odor of the HMO product. In some emoboduments, the antifoam agent is sterilisable by autoclaving or dry heat at certain conditions.

In some embodiments, the antifoam agent concentration in the HMO solution is from about 1 to about 100 g/kg (DS). In some embodiments, the antifoam agent concentration in the HMO solution is from about 1 to about 50 g/kg (DS). In some embodiments, the antifoam agent concentration in the HMO solution is from 3 to 45 g/kg (DS).

The concentration of the antifoam agent in the process solutions can be determined using, for example, commercially available test kits suitable for measuring soluble antifoam agents, such as the LCK 433 nonionic surfactants cuvette test (Hach Lange GmbH, Dusseldorf, Germany).

### Cloud point

As the temperature of an aqueous solution comprising an antifoam agent (particularly a non-ionic surfactant antifoam agent) is increased, a temperature is reached at which the solution appears cloudy. The minimum temperature at which the aqueous antifoam agent solution becomes cloudy (or turbid) is referred to as the "cloud point". Antifoam agent cloud points typically range from 0°C to 100°C, and are generally limited by the freezing and boiling points of water. The presence of other components, like salts, in a solution can depress or increase the antifoam agent's cloud point.

The cloud point of antifoam agents can be measured with a turbidimeter. An antifoam agent solution exhibiting a cloud point is usually visibly turbid at temperatures greater than the cloud point temperature. Turbidity can be measured and used as an indication of presence of antifoam agent and other precipitating components.

Cloud point temperature is separately determined for each antifoam agent in each different HMO solution. This is done by measuring the turbidity of a HMO solution containing antifoam agent at different temperatures.

In general, the antifoam agent used in a process described in this specification comprises an antifoam agent that exhibits a cloud point. The fermenter temperature during fermentation may be greater or less than the cloud point of the antifoam agent. In general, the fermentation temperature is selected based on the requirements of the product to be produced and the microorganism used in the fermentation. In some embodiments, the fermenter temperature during an HMO fermentation (*e.g.,* a 2'-FL fermentation or a 3-FL fermentation) is from about 35 to about 39°C. In general, an HMO fermentation is maintained at less than 50°C. In some embodiments, the fermentation temperature is a factor used in choosing a suitable antifoam agent.

In some embodiments, the cloud point temperature of the selected antifoam agent is greater than the fermentation temperature (or greater than the temperature used during at least a portion of the fermentation). In some embodiments, use of a fermentation temperature that is less than the cloud point temperature of the antifoam agent makes the antifoam agent more effective during the fermentation.

In some embodiments, the cloud point temperature of the selected antifoam agent is less than the fermentation temperature (or less than the temperature used during at least a portion of the fermentation). In some embodiments, use of a fermentation temperature that is greater than the cloud point temperature of the antifoam agent makes the antifoam agent more effective during the fermentation.

### Antifoam agent removal

Following fermentation, it is generally desired to substantially remove the antifoam agent from the HMO product. In some embodiments, at least about 80% of the antifoam agent present in the HMO fermentation product solution is removed. In some embodiments, at least 96% of the antifoam agent present in the HMO fermentation product solution is removed. In some embodiments, at least 97% of the antifoam agent present in the HMO fermentation product solution is removed. In some embodiments, at least 98% of the antifoam agent present in the HMO fermentation product solution is removed. In some embodiments, at least 99% of the antifoam agent present in the HMO fermentation product solution is removed.

Cell biomass may be removed from the fermentation broth using, for example, ultrafiltration and/or centrifugation. In some embodiments, one or more purification and/or concentration steps are carried out after cell removal. Such purification and/or concentration steps may include, for example, nanofiltration, anionic ion exchange, cationic ion exchange, activated carbon treatment, gel-filtration chromatography, electrodialysis, evaporation, crystallization or spray drying.

In some embodiments, the process comprises an antifoam agent removal step before removal of cell biomass (*e.g.,* before ultrafiltration). In some embodiments, the process comprises an antifoam agent removal step after removal of cell biomass (*e.g.,* after ultrafiltration).

In some embodiments, the process comprises an antifoam agent removal step before a nanofiltration step. In some embodiments, the process comprises an antifoam agent removal step after a nanofiltration step. In some embodiments, the process comprises antifoam agent removal step before and after a nanofiltration step.

In some embodiments, antifoam agent is precipitated during an antifoam agent removal step at a temperature that is at least the cloud point temperature of the antifoam agent. In some embodiments, antifoam agent is precipitated during an antifoam agent removal step at a temperature that is from 0°C to 10°C greater than the cloud point temperature of the antifoam agent. In some embodiments, antifoam agent is precipitated during an antifoam agent removal step at a temperature that is from 0°C to 5°C greater than the cloud point temperature of the antifoam agent. In some embodiments, antifoam agent is precipitated during an antifoam agent removal step at a temperature that is from 0°C to 2°C greater than the cloud point temperature of the antifoam agent. In some embodiments, antifoam agent is precipitated during an antifoam agent removal step at a temperature that is from 0°C to 1°C greater than the cloud point temperature of the antifoam agent. In some embodiments, antifoam agent is precipitated during an antifoam agent removal step at a temperature that is approximately equal to the cloud point temperature of the antifoam agent.

In some embodiments, the temperature at which the antifoam agent removal is conducted is within about 5°C of the temperature at which the HMO solution exhibits maximum turbidity. In some embodiments, the temperature at which the antifoam agent removal is conducted is within 4°C of the temperature at which the HMO solution exhibits maximum turbidity. In some embodiments, the temperature at which the antifoam agent removal is conducted is within 3°C of the temperature at which the HMO solution exhibits maximum turbidity. In some embodiments, the temperature at which the antifoam agent removal is conducted is within 2°C of the temperature at which the HMO solution exhibits maximum turbidity. In some embodiments, the temperature at which the antifoam agent removal is conducted is within 1°C of the temperature at which the HMO solution exhibits maximum turbidity. In some embodiments, the temperature at which the antifoam agent removal is conducted is at about the temperature at which the HMO solution exhibits maximum turbidity.

In some embodiments, the antifoam removal is conducted at a temperature of from 36 to 65°C. In some embodiments, the antifoam removal is conducted at a temperature of from 40 to 65°C. In some embodiments, the antifoam removal is conducted at a temperature of from 36 to 60°C. In some embodiments, antifoam removal is conducted at a temperature of from 43 to 55°C. In some embodiments, antifoam removal is conducted at a temperature of from 50 to 55°C. In some embodiments, antifoam removal is conducted at a temperature of about 50°C. In some embodiments, In some embodiments, antifoam removal is conducted at a temperature of about 55°C.

In some embodiments, after most of the antifoam agent has been precipitated, precipitated antifoam agent is separated from the HMO solution using, for example, filtration and/or centrifugation. In some embodiments, a filtration aid is used to further enable separation. Such a filtration aid may include, for example, cellulose, activated carbon, diatomaceous earth or a combination of any of the foregoing. In some embodiments, cellulose is used as a filtration aid. In some embodiments, diatomaceous earth is used as a filtration aid. In some embodiments, activated carbon is used as a filtration aid. In some embodiments, activated carbon powder is used as a filtration aid.

Filtration aids maybe mixed in the HMO solution containing antifoam agent before the filtration of the solution. In some embodiments, the amount of filtration aid used is from 0.5 to 3% (by weight) of the feed liquid. In some embodiments, the amount of filtration aid used is from 0.5 to 2% (by weight) of the feed liquid. In some embodiments, the amount of filtration aid used is from 0.5 to 1% (by weight) of the feed liquid. In some embodiments, the amount of filtration aid used is about 0.5% (by weight) of the feed liquid. In some embodiments, the amount of filtration aid used is about 1% (by weight) of the feed liquid. In some embodiments, the amount of filtration aid used is about 2% (by weight) of the feed liquid.

In some embodiments, the filtration aid is applied also as a precoat on the filter. In some embodiments, from 1 to 2 kg/m² of the filtration aid is applied as a precoat on the filter. In some embodiments, from 1 to 1.5 kg/m² of the filtration aid is applied as a precoat on the filter. In some embodiments, from 1.5 to 2 kg/m² of the filtration aid is applied as a precoat on the filter. In some embodiments, about 1 kg/m² of the filtration aid is applied as a precoat on the filter. In some embodiments, about 1.5 kg/m² of the filtration aid is applied as a precoat on the filter. In some embodiments, about 2 kg/m² of the filtration aid is applied as a precoat on the filter.

Filtration equipment may be selected from, for example, a depth filter, a plate- and-frame filter or a candle filter. Also, centrifugal separators may be used.

In some embodiments, remaining soluble antifoam agent is removed using activated carbon powder.

In some embodiments, an antifoam agent removal step is repeated two or more times, particularly when the solution contains a large amount of antifoam agent. In some embodiments, the same filtration aid is used in the first antifoam agent removal step and in the subsequent antifoam agent removal step. In some embodiments, one filtration aid is used in the first antifoam agent removal step and another filtration aid is used in the subsequent antifoam agent removal step.

In some embodiments, the point(s) during the HMO purification process at which antifoam agent is removed is/are selected based on environmental and economic considerations, e.g., avoiding additional heating and/or cooling steps.

In some embodiments, antifoam agent is removed from an intermediate process liquor, such as from a permeate of a membrane filtration, a concentrate of a membrane filtration, an ion exchanged solution, a crystallization mother liquor or the like. In additional or alternative embodiments, antifoam agent is removed from a final product. A final HMO product may be, for example, a syrup, spray dried powder or crystalline product. In some instances when the antifoam agent is removed from a crystalline or spray dried product, the product is dissolved, and the resulting solution is then heated to a temperature greater than the antifoam agent's cloud point to form a precipitate, which, in turn, is removed by filtering, optionally using a filtration aid, such as, for example, diatomaceous earth, activated carbon or cellulose.

In some embodiments, the antifoam agent is removed before an ion exchange step to reduce fouling of the ion exchange resin. If activated carbon is used for the removal of antifoam agent, the resulting process solution will typically contain less color, which can be helpful during the subsequent ion exchange step.

In some embodiments, the antifoam agent is removed after a nanofiltration step. In such an instance, the salt content of the process liquor will typically be less than the salt content before the nanofiltration step, and the dry substance content will typically be higher. A lesser salt content results better and easier filtration of the antifoam agent during the antifoam agent removal. Concentrated nanofiltered process solution has less volume, which, in turn, typically translates into less cost for filtration to remove the antifoam agent.

In some embodiments, antifoam agent is precipitated and removed from the HMO solution as discussed herein, and then the HMO solution is subjected to an evaporation step, which also includes again heating to a temperature greater than the cloud point temperature of the antifoam agent.

During the HMO manufacturing and purification process, unwanted premature precipitation of the antifoam agent can generally be minimized by processing the HMO solution at temperatures that are less than the cloud point temperature of the antifoam agent. In some embodiments, the process temperature in the various process steps before removal of antifoam agent is maintained at from about 1 to about 25°C. In some embodiments, the process temperature of the various process steps before removal of antifoam agent is maintained at from 5 to 20°C. In some embodiments, the process temperature of the various process steps before removal of antifoam agent is maintained at from 5 to 15°C. In some embodiments, the process temperature of the various process steps before removal of antifoam agent is maintained at from 5 to 10°C. In some embodiments, the process temperature of the various process steps before removal of antifoam agent is maintained at from 5 to 8°C. In some embodiments, the process temperature of the various process steps before removal of antifoam agent is maintained at less than 10°C. In some embodiments, the process temperature of the various process steps before removal of antifoam agent is maintained at less than 8°C. In some embodiments, the process temperature of the various process steps before removal of antifoam agent is maintained at less than 7°C. In some embodiments, the process temperature of the various process steps before removal of antifoam agent is maintained at less than 6°C. In some embodiments, the process temperature of the various process steps before removal of antifoam agent is maintained at less than 5°C.

In some embodiments, the dry substance concentration in the HMO solution containing antifoam agent is from 3 to 30 g/100 g when the solution is fed into an antifoam agent removal step described herein. In some embodiments, the dry substance concentration in the HMO solution containing antifoam agent is from 3 to 20 g/100 g when the solution is fed into an antifoam agent removal step described herein. In some embodiments, the dry substance concentration in the HMO solution containing antifoam agent is from 10 to 30 g/100 g when the solution is fed into an antifoam agent removal step described herein. In some embodiments, the dry substance concentration in the HMO solution containing antifoam agent is from 14 to 30 g/100 g when the solution is fed into an antifoam agent removal step described herein.

In some embodiments, at least 80% of the antifoam agent in the HMO solution is removed. In some embodiments, at least 90% of the antifoam agent in the HMO solution is removed. In some embodiments, at least 96% of the antifoam agent in the HMO solution is removed. In some embodiments, at least 98% of the antifoam agent in the HMO solution is removed. In some embodiments, at least 99% of the antifoam agent in the HMO solution is removed.

In some embodiments, the antifoam agent concentration in the HMO solution after the antifoam agent removal step is from about 5 to about 12,500 mg/kg (DS). In some embodiments, the antifoam agent concentration in the HMO solution after the antifoam agent removal step is from 6 to 12,200 mg/kg (DS). In some embodiments, the antifoam agent concentration in the HMO solution after the antifoam agent removal step is less than 12,500 mg/kg (DS). In some embodiments, the antifoam agent concentration in the HMO solution after the antifoam agent removal step is less than 12,200 mg/kg (DS). In some embodiments, the antifoam agent concentration in the HMO solution after the antifoam agent removal step is less than 12,000 mg/kg (DS). In some embodiments, tthe antifoam agent concentration in the HMO solution after the antifoam agent removal step is less than 10,000 mg/kg (DS). In some embodiments, the antifoam agent concentration in the HMO solution after the antifoam agent removal step is less than 7,000 mg/kg (DS). In some embodiments, the antifoam agent concentration in the HMO solution after the antifoam agent removal step is less than 6,000 mg/kg (DS). In some embodiments, the antifoam agent concentration in the HMO solution after the antifoam agent removal step is less than 5,000 mg/kg (DS). In some embodiments, the antifoam agent concentration in the HMO solution after the antifoam agent removal step is less than 4,000 mg/kg (DS). In some embodiments, the antifoam agent concentration in the HMO solution after the antifoam agent removal step is less than 3,000 mg/kg (DS). In some embodiments, the antifoam agent concentration in the HMO solution after the antifoam agent removal step is less than 2,000 mg/kg (DS). In some embodiments, the antifoam agent concentration in the HMO solution after the antifoam agent removal step is less than 1,000 mg/kg (DS). In some embodiments, the antifoam agent concentration in the HMO solution after the antifoam agent removal step is less than 900 mg/kg (DS). In some embodiments, the antifoam agent concentration in the HMO solution after the antifoam agent removal step is less than 800 mg/kg (DS). In some embodiments, the antifoam agent concentration in the HMO solution after the antifoam agent removal step is less than 600 mg/kg (DS). In some embodiments, the antifoam agent concentration in the HMO solution after the antifoam agent removal step is less than 400 mg/kg (DS). In some embodiments, the antifoam agent concentration in the HMO solution after the antifoam agent removal step is less than 200 mg/kg (DS). In some embodiments, the antifoam agent concentration in the HMO solution after the antifoam agent removal step is less than 100 mg/kg (DS). In some embodiments, the antifoam agent concentration in the HMO solution after the antifoam agent removal step is less than 50 mg/kg (DS). In some embodiments, the antifoam agent concentration in the HMO solution after the antifoam agent removal step is less than 10 mg/kg (DS).

In some embodiments, the HMO solution is cooled to a temperature of less than 20°C after removal of the antifoam agent as disclosed herein. In some embodiments, the HMO solution is cooled to a temperature of less than 15°C after removal of the antifoam agent as disclosed herein. In some embodiments, the HMO solution is cooled to a temperature of less than 10°C after removal of the antifoam agent as disclosed herein. In some embodiments, the HMO solution is cooled to a temperature of less than 5°C after removal of the antifoam agent as disclosed herein.

In some embodiments, an antifoam agent removal step from an HMO solution as disclosed in this specification does not substantially affect (*e.g.,* change by greater than about 1%) the area% of the carbohydrates (*e.g.,* sugars) of the HMO solution.

### Additional treatments

In some embodiments, the process comprises subjecting the HMO solution to one or more of the following treatments: an enzymatic treatment (*e.g.,* enzymatic hydrolysis of lactose), nanofiltration, electrodialysis, chromatography, ion exchange (e.g., cation ion exchange, anion ion exchange or both), evaporation, activated carbon, crystallization, evaporation and/or spray-drying.

The additional treatments may typically be carried out in various orders, as well as being repeated at different points in the process. In some embodiments, the process comprises a combination of at least three of the above additional treatments. In some embodiments, the process comprises a combination of at least four of the above additional treatments.

In some embodiments, the process comprises at least one cation exchange step. In some embodiments, the process comprises at least one anion exchange step. In some embodiments, the process comprises both a cation exchange step and an anion exchange step.

In some embodiments, the HMO solution is subjected to evaporation. This can be helpful, for example, to concentrate the HMO by removing a solvent (*e.g.,* water). In some embodiments, evaporation is the final purification step of the desired HMO.

In some embodiments, the HMO solution is subjected to spray drying. In some embodiments, the spray-drying is carried out under conditions discussed in WO2019/160922 (incorporated by reference into this specification). In some embodiments, the HMO feed into the spray dryer has a Brix value of from about 8 to about 75%Brix. In some embodiments, the Brix value is from about 30 to about 65%Brix. In some embodiments, the Brix value is from about 50 to about 60%Brix. In some embodiments, the feed into the spray dryer is at a temperature of from about 2 to about 70°C immediately before being dispersed into droplets in the spray dryer. In some embodiments, the feed into the spray dryer is at a temperature of from about 30 to about 60°C immediately before being dispersed into droplets in the spray dryer. In some embodiments, the feed into the spray dryer is at a temperature of from about 2 to about 30°C immediately before being dispersed into droplets in the spray dryer In some embodiments, the spray drying is carried out at a temperature of from about 20 to about 90°C. In some embodiments, spray-drying is the final purification step for the desired HMO.

In some embodiments, the process comprises crystallization. In some embodiments, no organic solvent is used during the crystallization. In some embodiments, the crystallization comprises a crystallization process disclosed in WO2018/164937 (incorporated by reference into this specification). In some embodiments, crystallization is the final purification step of the desired HMO. In some embodiments, the process comprises both crystallization and evaporation. In some embodiments, the process comprises both crystallization and spray-drying.

### EXAMPLES

The following examples are merely illustrative, and not limiting to the remainder of this specification in any way.

Turbidities of the HMO solutions were measured at room temperature.

Antifoam agent concentrations in the HMO solutions were measured at room temperature.

### Example 1

### Antifoam agent removal of 2-FL solution

A 2'-FL solution, which contained STRUKTOL J673 as antifoam agent, was used as feed. ARBOCEL B-800 powdered cellulose (JRS Pharma GmbH & Co. KG, Rosenberg, Germany) and NORIT DX1 activated carbon (Cabot Switzerland GmbH, Schaffhausen, Switzerland) were used as filtration aids for antifoam agent removal. The feed material was heated to 55°C in a jacketed reactor vessel with stirring. The filtration aid (1% of the feed liquid weight) was added to the reactor. The resulting mixture was incubated at 55°C with stirring for about 1 hr. After precoating a SEITZ depth filter (Pall Corp., Hampshire, United Kingdom) with the filtration aid (1.5 kg/m²), the mixture was circulated through the depth filter for 0.5 hr, and the resulting filtrate was then collected. The antifoam agent concentration was measured from all samples with a HACH LCK 433.

The properties of the 2'-FL solution before antifoam removal are shown in **Table 1-1.**

**TABLE 1-1**

| **Properties of 2'-FL solution before antifoam removal** | |
|---|---|
| Dry substance, g/100 g | 29.1 |
| pH | 6.35 |
| Conductivity, mS/cm | 8.0 |
| Color, ICUMSA | 65,500 |
| Antifoam conc, mg/kg (DS) | 26,000 |
| Turbidity, NTU | >800 |
| 2'-FL, area-% | 83.0 |
| LDFT, area-% | 7.7 |
| Other carbohydrates, area-% | 9.3 |

And the properties of the 2'-FL solution after the antifoam agent removal are shown in **Table 1-2.**

**TABLE 1-2**

| **Properties of 2'-FL solution after antifoam removal** | | |
|---|---|---|
| | **ARBOCEL treated soln** | **NORIT DX1 treated soln** |
| Dry substance, g/100 g | 16.2 | 15.3 |
| pH | 6.40 | 6.46 |
| Conductivity, mS/cm | 6.9 | 6.6 |
| Color, ICUMSA | 65,300 | 16,600 |
| Antifoam conc, mg/kg (DS) | 4,600 | 300 |
| Turbidity, NTU | 11 | 4 |
| 2'-FL, area-% | 83.2 | 83.1 |
| LDFT, area-% | 7.7 | 7.7 |
| Other carbohydrates, area-% | 9.1 | 9.2 |

As can be seen, when ARBOCEL B-800 powdered cellulose treatment was used, 82.3% of the antifoam agent was removed. And when NORIT DX1 activated carbon treatment was used, 98.8% of the antifoam agent was removed. NORIT DX1 treatment also reduced the color. In both instances, the antifoam agent removal did not substantially affect the HPLC area percentages of the HMOs.

### Example 2

### Antifoam agent removal of 2-FL solution

A 2'-FL solution, which contained STRUKTOL J673 as antifoam agent, was used as feed. NORIT DX1 activated carbon was used as the filtration aid for the antifoam agent removal. The feed material was heated to 50-55°C with stirring. The filtration aid (1% of the feed liquid weight) was added to the reactor. The resulting mixture was incubated at 50-55°C with stirring for about 1 hr. The mixture was then circulated through a SEITZ depth filter for about 0.5 hr at 55°C, and the resulting filtrate was then collected. The antifoam agent concentration was measured from the feed sample with a HACH LCK 433, and from the product with a HACH LCK 333.

Properties of 2'-FL solution before the antifoam agent removal are shown in **Table 2-1.**

**TABLE 2-1**

| **Properties of 2'-FL solution before antifoam removal** | |
|---|---|
| Dry substance, g/100 g | 14.1 |
| pH | 3.58 |
| Conductivity, mS/cm | 1.8 |
| Color, ICUMSA | 45,000 |
| Antifoam conc, mg/kg (DS) | 39,800 |
| 2'-FL, area-% | 75.3 |
| LDFT, area-% | 7.3 |
| Other carbohydrates, area-% | 17.4 |

And properties of 2'-FL solution after antifoam agent removal are shown in **Table 2-2.**

**TABLE 2-2**

| **Properties of 2'-FL solution after antifoam removal** | |
|---|---|
| Dry substance, g/100 g | 13.3 |
| pH | 3.75 |
| Conductivity, mS/cm | 2.2 |
| Color, ICUMSA | 3,500 |
| Antifoam conc, mg/kg (DS) | 800 |

With the NORIT DX1 activated carbon treatment, 98.0% of the antifoam agent was removed. In addition, 92.2% of the color was removed.

### Example 3

### Turbidity removal of 2-FL solution

2'-FL solution, which contained STRUKTOL J673 as antifoam agent, was used as feed. KENITE 300 diatomaceous earth (Imerys, Paris, France), NORIT DX1 activated carbon and ARBOCEL B-800 powdered cellulose were used as filtration aids. The feed material was heated to 50°C with stirring. The effect of each filtration aid was tested by adding it as body feed into the 2'-FL solution and then incubating the mixture at 50°C with stirring for about 1 hr. In each instance, the filtration was conducted using a SEITZ depth filter precoated with the filtration aid being tested.

Properties of the 2'-FL solution before antifoam removal are shown in **Table 3-1.**

**TABLE 3-1**

| **Properties of the 2'-FL solution before antifoam removal** | |
|---|---|
| Dry substance, g/100 g | 16.26 |
| pH | 6.32 |
| Conductivity, mS/cm | 5.63 |
| Color (unfiltered), ICUMSA | 56,900 |
| Color (filtered), ICUMSA | 54,200 |
| Turbidity at 5 °C, NTU | 43.7 |
| Turbidity at 40 °C, NTU | > 1,000 |
| 2'-FL, area-% | 77.0 |
| LDFT, area-% | 7.5 |
| Other carbohydrates, area-% | 15.5 |

The properties of 2'-FL solutions after each antifoam removal treatment are shown in **Table 3-2.**

**TABLE 3-2**

| **Properties of the 2'-FL solution after antifoam removal** | | | | |
|---|---|---|---|---|
| **Filtration aid** | **KENITE 300** | **NORIT DX1** | **NORIT DX1** | **ARBOCEL** |
| Bodyfeed,%/lw | 1 | 1 | 2 | 1 |
| Precoat, kg/m² | 1 | 2 | 2 | 2 |
| Dry substance, g/100 g | 14.1 | 14.0 | 13.6 | 19.2 |
| pH | 6.38 | 6.42 | 6.47 | 6.30 |
| Conductivity, mS/cm | 5.90 | 5.94 | 5.98 | 6.88 |
| Color (unfiltered), ICUMSA | 59,800 | 13,400 | 6,100 | 58,000 |
| Color (filtered), ICUMSA | 59,300 | 13,000 | 6,000 | 57,600 |
| Turbidity at 5°C, NTU | 85 | 5 | 2 | 12 |
| Turbidity at 40°C, NTU | 146 | 6 | 2 | 353 |

### Example 4

### Antifoam agent removal of 2-FL solution

A 2'-FL solution, which contained STRUKTOL J673A as antifoam agent, was used as feed. NORIT DX1 activated carbon and CLARCEL CBR3 diatomaceous earth (Chemviron, Feluy, Belgium) were used as filtration aids. The feed material was heated to 50°C with stirring. The effect of each filtration aid was tested by adding it as body feed into the feed material and then incubating the mixture at 50°C with stirring for about 1 hr. Filtration was conducted using a SEITZ depth filter precoated with the filtration aid being used. The material was circulated through the filter for about 0.5 hr, and then the resulting filtrate was collected. The antifoam agent concentration was measured from all samples with a HACH LCK 433.

Properties of 2'-FL solution before antifoam removal are shown in **Table 4-1.**

**TABLE 4-1**

| **Properties of the 2'-FL solution before antifoam removal** | |
|---|---|
| Dry substance, g/100 g | 3.96 |
| pH | 3.5 |
| Conductivity, mS/cm | 0.12 |
| Antifoam conc, mg/kg (DS) | 16,300 |
| Turbidity, NTU | 313 |

The properties of 2'-FL solutions after each treatment are shown in **Table 4-2.**

**TABLE 4-2**

| **Properties of the 2'-FL solution after antifoam removal** | | |
|---|---|---|
| **Filtration aid** | **NORIT DX1** | **CLARCEL CBR3** |
| Bodyfeed,%/lw | 1 | 0.5 |
| Precoat, kg/m² | 2 | 2 |
| Dry substance, g/100 g | 2.17 | 2.50 |
| pH | 5.5 | 4.2 |
| Conductivity, mS/cm | 0.03 | 0.05 |
| Antifoam conc, mg/kg (DS) | 500 | 12,200 |
| Turbidity, NTU | 1 | 9 |

With NORIT DX1 activated carbon being used as a filtration aid, 96.9% of antifoam agent and 99.7% of turbidity were removed. With CLARCEL CBR3 diatomaceous earth being used as a filtration aid, 25.2% of antifoam agent and 97.1% of turbidity were removed. It is believed that the filtration using the CLARCEL CBR3 removed essentially only insoluble antifoam, which resulted in a filtrate still having a large amount of remaining antifoam agent. The filtration using the NORIT DX1, in contrast, is believed to have removed both insoluble and soluble antifoam, resulting in a filtrate containing much less remaining antifoam agent.

### Example 5

### Antifoam agent removal of 2-FL solution

A 2'-FL solution, which contained STRUKTOL J673 as antifoam agent, was used as feed. NORIT DX1 and GBSP activated carbon were used as filtration aids for antifoam agent removal. The feed material was heated to 55°C in a jacketed reactor vessel with stirring. Filtration aid (1% of the feed liquid weight) was added to the reactor. The resulting mixture was then incubated at 55°C with stirring for about 1 hr. The solution was subsequently circulated through a FUNDABAC candle filter (DrM, Maennedorf, Switzerland) for 1 hr, and then the resulting filtrate was collected. The filtrate was directed back to the feed vessel. In each instance, the filter was not precoated with the filtration aid.

Properties of 2'-FL solution before antifoam removal are shown in **Table 5-1.**

**TABLE 5-1**

| **Properties of 2'-FL Solution before antifoam removal** | |
|---|---|
| Dry substance, g/100 g | 22.3 |
| pH | 5.8 |
| Conductivity, mS/cm | 5.2 |
| Color, ICUMSA | 27,800 |
| Antifoam conc, mg/kg (DS) | 41,500 |
| 2'-FL purity, area-% | 91.60 |
| LDFT purity, area-% | 4.6 |
| Lactose purity, area-% | 3.3 |
| Other carbohydrates, purity, area-% | 0.5 |

The properties of 2'-FL solutions after antifoam agent removal are shown in **Table 5-2.**

**TABLE 5-2**

| **Properties of the 2'-FL solution after antifoam removal** | | |
|---|---|---|
| | **DX1 treated 2'FL solution** | **GBSP treated 2'FL solution** |
| Dry substance, g/100 g | 20.7 | 21.4 |

| | **DX1 treated 2'FL solution** | **GBSP treated 2'FL solution** |
|---|---|---|
| pH | - | 5.7 |
| Conductivity, mS/cm | - | 5.5 |
| Color, ICUMSA | 8,000 | 13,000 |
| Antifoam conc, mg/kg (DS) | 770 | 6,300 |

As can be seen, with the NORIT GBSP activated carbon filtration aid, 84.7% of the antifoam agent was removed. And with the NORIT DX1 activated carbon filtration aid, 98.1% of the antifoam agent was removed. Use of the NORIT DX1 activated carbon filtration aid also reduced more color.

To reduce antifoam agent and color even more, a second antifoam removal was conducted, again using NORIT DX1 and GBSP as filtration aids. The treatment was carried with the same parameters as with the first filtration. The results are shown in **Table 5-3.**

**TABLE 5-3**

| **Properties of the 2'-FL solution after two antifoam removal steps** | | |
|---|---|---|
| | **2 x DX1 treated 2'FL solution** | **2 x GBSP treated 2'FL solution** |
| Dry substance, g/100 g | 20.9 | 20.8 |
| pH | 5.97 | 5.7 |
| Conductivity, mS/cm | 5.5 | 5.6 |
| Color, ICUMSA | 3,100 | 5,000 |
| Antifoam conc, mg/kg (DS) | 120 | 90 |

With two antifoam removal steps using NORIT DX1 activated carbon as a filtration aid, 99.7% of the antifoam agent was removed. And with two antifoam removal steps using NORIT GBSP as a filtration aid, 99.8% of the antifoam agent was removed.

### Example 6

### Antifoam agent removal of 2-FL solution

A 2'-FL solution, which contained STRUKTOL J673 as antifoam agent, was used as feed. NORIT DX1 activated carbon was used as a filtration aid for antifoam agent removal. The feed material was heated to 50°C in a jacketed reactor vessel with stirring. The filtration aid (1.15% of the feed liquid weight) was added to the reactor. The resulting mixture was incubated at 50°C with stirring for about 1 hr. The mixture was then circulated through a plate and frame filter (SEITZ depth filter, without any precoating with the filter aid) for 40 min, and then the resulting filtrate was collected. The filtrate was directed back to the feed vessel, and then the activated carbon treatment was repeated.

Properties of 2'-FL solution are shown in **Table 6-1.**

**TABLE 6-1**

| | **Initial feed** | **1 x DX1 treated filtrate** | **2 x DX1 treated filtrate** |
|---|---|---|---|
| Dry substance, g/100 g | 17.20 | 15.7 | 13.84 |
| pH | 5.85 | 6.20 | 6.43 |
| Conductivity, mS/cm | 6.7 | 6.5 | 6.1 |
| Color, ICUMSA | 49,217 | 23,416 | 8,480 |
| Turbidity, NTU | >1,000 | 241 | 10 |
| Antifoam conc, mg/kg (DS) | 32,740 | 1,587 | 387 |
| 2'-FL purity, area-% | 80.36 | 80.24 | 80.02 |
| LDFT purity, area-% | 9.99 | 10.05 | 10.18 |
| Lactose purity, area-% | 7.77 | 15.70 | 13.84 |

With the first NORIT DX1 activated carbon treatment, 95% of the antifoam agent was removed. With the second NORIT DX1 treatment, 99% of the antifoam agent was removed. The turbidity was greater than the measuring range (>1,000 NTU) in the feed. The first NORIT DX1 activated carbon treatment reduced the turbidity to 241 NTU, and the second treatment reduced the turbidity to 10 NTU. The first NORIT DX1 activated carbon treatment reduced the color content from 49,200 ICUMSA to 23,400 ICUMSA, and the second treatment reduced the color content to 8,500 ICUMSA. The treatments did not substantially impact the HMO area% profile, pH or conductivity.

### Example 7

### Antifoam agent removal of 3-FL solution

A 3-FL solution, which contained STRUKTOL J673A as antifoam agent, was used as feed. NORIT DX1 activated carbon was used as filtration aid for the antifoam agent reduction. The following antifoam agent reduction procedure was conducted twice. The feed material was heated to 55°C in a jacketed reactor vessel with stirring. The filtration aid (2% of the feed liquid weight) was then added to the reactor. The resulting mixture was incubated at 55°C with stirring for about 1 hr. After incubation, the mixture was circulated through a SEITZ depth filter for about 0.5 hr, and then the resulting filtrate was collected. The antifoam agent concentration was measured from the feed with a HACH LCK 433, and from the product with a HACH LCK 333.

Properties of the 3-FL solution before antifoam removal are shown in **Table 7-1.**

**TABLE 7-1**

| **Properties of 3-FL solution before antifoam removal** | |
|---|---|
| Dry substance, g/100 g | 17.6 |
| pH | 6.48 |
| Conductivity, mS/cm | 28.4 |
| Color, ICUMSA | 139,800 |
| Antifoam conc, mg/kg (DS) | 3,200 |
| 3-FL, area-% | 95.0 |
| Other carbohydrates, area-% | 5.0 |

The properties of 3-FL Nanofiltration concentrate after antifoam agent reduction are shown in **Table 7-2.**

**TABLE 7-2**

| **Properties of 3-FL Nanofiltration concentrate after antifoam removal** | |
|---|---|
| Dry substance, g/100 g | 13.0 |
| pH | 6.61 |
| Conductivity, mS/cm | 26.4 |
| Color, ICUMSA | 9,000 |
| Antifoam conc, mg/kg (DS) | 6 |
| 3-FL, area-% | 95.9 |
| Other carbohydrates, area-% | 4.1 |

As can be seen, 99.8% of the antifoam agent was removed. In addition, 93.6% of the color was removed.

### Example 8

### Turbidity curves of different antifoam agent agents

Turbidity (NTU) measured from different HMO solutions from HMO fermentations using STRUKTOL J673, FOAM BLAST 882 or STRUKTOL J673A are shown in **Table 8-1** and **Figure 1****.**

**TABLE 8-1**

| **Sample** | **Temp (°C)** | **Turbidity (NTU)** |
|---|---|---|
| STRUKTOL J673 | 4 | 10 |
| STRUKTOL J673 | 18 | 1 |
| STRUKTOL J673 | 23 | 1.21 |
| STRUKTOL J673 | 26 | 19 |
| STRUKTOL J673 | 28 | 41 |
| STRUKTOL J673 | 29 | 60 |
| STRUKTOL J673 | 31 | 65 |
| STRUKTOL J673 | 33 | 71 |
| STRUKTOL J673 | 36 | 94 |
| STRUKTOL J673 | 41 | 87 |
| STRUKTOL J673 | 43 | 115 |
| STRUKTOL J673 | 50 | 134 |
| STRUKTOL J673 | 55 | 125 |
| STRUKTOL J673 | 60 | 86 |
| FOAM BLAST | 4 | 0.7 |
| FOAM BLAST | 18 | 0.6 |
| FOAM BLAST | 23 | 12 |
| FOAM BLAST | 26 | 113 |
| FOAM BLAST | 28 | 222 |
| FOAM BLAST | 29 | 266 |
| FOAM BLAST | 31 | 262 |
| FOAM BLAST | 33 | 306 |
| FOAM BLAST | 36 | 346 |
| FOAM BLAST | 41 | 279 |
| FOAM BLAST | 43 | 314 |
| FOAM BLAST | 50 | 391 |
| FOAM BLAST | 55 | 282 |
| FOAM BLAST | 60 | 197 |
| STRUKTOL J673A | 14 | 1.21 |
| STRUKTOL J673A | 21 | 1.06 |
| STRUKTOL J673A | 24 | 10.2 |
| STRUKTOL J673A | 26 | 55.4 |
| STRUKTOL J673A | 28 | 47.2 |
| STRUKTOL J673A | 31 | 81.7 |
| STRUKTOL J673A | 43 | 198 |
| STRUKTOL J673A | 48 | 150 |
| STRUKTOL J673A | 55 | 89 |

## Claims

1. A process for preparing a purified human milk oligosaccharide (HMO) derived from a fermentation process, wherein the process comprises:
(a) precipitating an antifoam agent in a solution comprising the HMO and the antifoam agent by heating the solution to a precipitation temperature of from 36 to 65°C, wherein the precipitation temperature is within 5°C of a temperature at which the HMO solution exhibits maximum turbidity; and
(b) filtering precipitated antifoam agent from the HMO solution at the precipitation temperature to form a filtrate comprising the HMO.

2. A process according to claim 1, wherein the HMO solution is maintained at a temperature of less than the cloud point temperature of the antifoam agent between completion of the fermentation process and Step (a).

3. The process according to any one of the preceding claims, wherein the HMO solution is maintained at from 5 to 15°C between completion of the fermentation process and Step (a).

4. The process according to any one of the preceding claims, wherein the antifoam agent is precipitated at within 5°C of the cloud point temperature of the antifoam agent during Step (a).

5. The process according to any one of the preceding claims, wherein a portion of the antifoam agent is precipitated at a temperature of from 36 to 65°C or from 36 to 60°C or from 40 to 65°C during Step (a).

6. The process according to any one of the preceding claims, wherein:
the heated HMO solution of Step (a) further comprises a filtration aid when it is filtered, and
the filtration aid is selected from activated carbon, cellulose and diatomaceous earth.

7. The process according to any one of the preceding claims, wherein greater than 90% of the antifoam agent in the HMO solution at the completion of the fermentation is removed from the HMO solution.

8. The process according to any one of the preceding claims, wherein the antifoam agent is removed using at least two filtration steps that comprise use of a filtration aid.

9. The process according to any one of the preceding claims, wherein the HMO is 2'-fucosyllactose or 3-fucosyllactose.

10. The process according to any one of the previous claims, wherein the process further comprises separate cation ion exchange and anion ion exchange steps.

11. The process according to any one of the previous claims, wherein the process further comprises vacuum evaporation.

12. The process according to any one of the preceding claims wherein the concentration of the antifoam agent in the solution to be filtered is from about 1 g/kg to about 100 g/kg (DS).

13. The process according to any one of the preceding claims, wherein additional antifoam agent is removed from the HMO solution after the filtration of Step (b) by contacting the HMO solution with activated carbon.

14. The process according to claim 13, wherein the HMO solution is cooled to less than 20°C after being contacted with the activated carbon.

15. The process according to any one of the preceding claims, wherein the HMO solution is cooled to a temperature of less than 20°C after the filtration Step (b).
